# EUROPEAN PATENT APPLICATION

(11) **EP 4 286 113 A1**
(43) Date of publication of application: **06.12.2023**
(21) Application number: 21922130.6
(22) Date of filing: 28.05.2021
(51) Int. Cl.: B25J 17/00

(54) **ELECTRIC LOCKING ROTARY JOINT**

(30) Priority: 01.02.2021 CN 202110133995
(71) Applicant: XIANJU XINGZHI TECHNOLOGY CO., LTD., Taizhou, Zhejiang 317317 (CN)
(72) Inventor: MA, Jianqun, Beijing 100000 (CN)
(74) Representative: Hahner, Ralph
(86) International application number: PCT/CN2021/096563
(87) International publication number: WO 2022/160532

(57) **Abstract**

Some embodiments of the present invention provide an electric locking rotary joint, including a motor (1), a shaft joint and a ball joint, wherein the motor (1) is a double-output-shaft motor or a single-output-shaft motor, and corresponding to each motor shaft, a shaft joint shell (2) or a ball joint shell (3) is fixedly connected with the machine body of the motor (1); and a locking and loosening mechanism of the shaft joint or the ball joint is fixedly connected with the motor shaft, and locking and loosening of the shaft joint or the ball joint are controlled by means of the on/off and forward/reverse rotation of the motor (1). The rotary joint is flexible and convenient to control, and the system is simple; and meanwhile, since the motor (1) is connected in series between rod-shaped members, the volume and weight of the rotary joint are relatively small, so that the performance of a rod-shaped serial mechanism, and especially mechanisms such as an endoscope support can be obviously improved.

## Description

### Technical Field

The invention relates to key components of rod-shaped serial mechanisms, and specifically relates to an electric locking rotary joint.

### Background

A rod-shaped serial mechanism is widely used in occasions such as a mechanical arm, a multi-degree-of-freedom position adjusting device, an end effector displacement mechanism and the like, and the actions of the rod-shaped serial mechanism are mainly realized by various rotary joints in a serial rod system, so that the rotary joints are key components. When at work, the rotary joint needs to change the rotating or locking state according to working conditions, that is, the rotary joint is equipped with a controllable locking device. At present, there are various types of locking devices, such as forms disclosed in the Chinese Patent "Rotary Joint Electromagnetic Locking Device in 200510092591". However, along with the continuous miniaturization development of man-machine cooperation mechanical arms, master-slave mechanical arms, medical instruments, optical instruments and the like, the sizes and weights of the locking devices of various existing rotary joints are difficult to meet design indexes. The present invention provides an electric locking rotary joint by using a structure in which a motor drives a screw nut pair or a cam pair, which can meet the requirements of a novel rod-shaped serial mechanism for the rotary joint.

### Summary

Some embodiments of the present invention provide an electric locking rotary joint, which are small in size, light in weight, large in locking force, fast in locking-loosening conversion and simple in control, and are used for a novel rod-shaped serial mechanism, specifically relate to a medical endoscope support and other occasions.

This invention provides an electric locking rotary joint, mainly includes a motor, a shaft joint and a ball joint, the motor is a double-output-shaft motor or a single-output-shaft motor, corresponding to each motor shaft, the shaft joint shell or a ball joint shell is fixedly connected with the machine body of the motor, and meanwhile, a locking and loosening mechanism of the shaft joint or the ball joint is fixedly connected with the motor shaft.

In some embodiments, the shaft joint shell is rotatably sleeved on a rotating shaft, a axis of the rotating shaft is perpendicular to a axis of the motor shaft, a brake ring which includes an opening is sleeved on the rotating shaft, the two ends of the rotating shaft extend out of the shaft joint shell, the rotating shaft and the shaft joint shell form the shaft joint, the shaft joint shell is hollow and is referred to as a space T, the motor shaft is disposed in the space T, a screw is fixed on the motor shaft, a nut is disposed on the screw to form a screw pair, a rear end of a locking fork is fixedly connected with the nut, a front end of the locking fork abuts against the outer side of the opening of the brake ring, a baffle P is fixed on the shaft joint shell in the space T, one end face of a disc spring set P is pressed on the baffle P, and the other end face of the disc spring set P is pressed on the locking fork, so as to press the locking fork towards the brake ring.

In some embodiments, the ball joint shell is hollow and is referred to as a space L, the motor shaft is disposed in the space L, the space L includes a pore channel, and a segment of the pore channel includes an opening, a axis of the pore channel is perpendicular to a axis of the motor shaft, a segment of spherical surface is disposed on the opening of the pore channel, a spherical pressing block is disposed in the pore channel, the spherical surface of the spherical pressing block and the spherical surface at the outlet of the pore channel form a spherical space, a ball head is rotatably disposed in the spherical space, the ball head and the two spherical surfaces form the ball joint, a plate cam fixed on the motor shaft is disposed in the space L, a axis of the plate cam is parallel to the axis of the motor shaft, a flat-faced follower is in contact coupling with the plate cam, the flat-faced follower and the plate cam form a cam pair, the flat-faced follower is fixedly connected with the spherical pressing block, a baffle Q fixed with the ball joint shell is disposed in the space L, one end face of a disc spring set Q is pressed on the baffle Q, and the other end face of the disc spring set Q is pressed on the spherical pressing block, so as to press the spherical pressing block towards the ball head.

In some embodiments, the brake ring is an annular member with an opening, which is movably sleeved on the rotating shaft; two bosses which are outward are disposed on the opening, outer sides of the two bosses are inclined surfaces, the two inclined surfaces are symmetrical, the distance between the outer ends of the two inclined surfaces is small, and the distance between the inner ends is large to form an outer inclined surface group.

In some embodiments, the front end of the locking fork is a group of inner inclined surfaces, and the geometric dimension of the group of inner inclined surfaces enables the same to be attached to the outer inclined surface group on the bosses of the brake ring.

In some embodiments, the spherical diameter at the opening of the open pore channel in the space L, the spherical diameter of the spherical pressing block and the diameter of the ball head are the same.

In some embodiments, the motor is a servo motor or a stepper motor.

The working process of the electric locking rotary joint is as follows: at an initial state, the pressure of the disc spring set P pushes the locking fork, and the group of inner inclined surfaces on the front end of the locking fork is tightly pressed on the outer inclined surface group of the boss of the brake ring, so that the brake ring generates an elastic deformation of shrinking inwards to tightly hold the rotating shaft, therefore the rotating shaft cannot rotate relative to the shaft joint shell, and thus the shaft joint is in a locked state. When the motor rotates, the screw on the motor shaft rotates therewith, so that the nut moves, the locking fork fixed on the nut overcomes the thrust of the disc spring set P to move backwards, the inner inclined surface group on the front end of the locking fork leaves the outer inclined surface group of the boss of the brake ring, so that the elastic deformation of the brake ring disappears to loosen the rotating shaft, therefore the rotating shaft can rotate relative to the shaft joint shell, and thus the shaft joint enters a rotatable state. The motor rotates reversely, the locking fork returns to the original state, and the shaft joint is locked again.

At the initial state, the pressure of the disc spring set Q pushes the spherical pressing block, the spherical surface of the spherical pressing block presses the ball head on the spherical surface at the outlet of the ball joint shell, so as to tightly held the ball head, so that the ball head cannot rotate relative to the ball joint shell, and thus the ball joint is in the locked state. When the motor rotates, the cam on the motor shaft rotates therewith, a high point of the cam pushes the flat-faced follower to move, the spherical pressing block fixed on the flat-faced follower overcomes the thrust of the disc spring set Q to move backwards, the spherical surface of the spherical pressing block leaves the ball head to loosen the ball head, so that the ball head can rotate relative to the ball joint shell, and thus the ball joint enters the rotatable state. The motor rotates reversely, the high point of the cam leaves the flat-faced follower, the flat-faced follower and the spherical pressure block fixed therewith return to the original state under the thrust of the disc spring set Q, and the ball joint is locked again.

Therefore, the locking and loosening of the shaft joint or the ball joint can be conveniently controlled just by controlling the forward and reverse rotation of the motor.

Since the locking and loosening of the rotary joint are controlled by using the forward and reverse rotation of the motor, the control is flexible and convenient, and the system is greatly simplified. At the same time, since the motor is connected in series between rod-shaped members, the volume and weight of the rotary joint are relatively small, and the performance of the rod-shaped serial mechanism, especially an endoscope support and other mechanisms can be obviously improved by using the rotary joint.

### Brief Description of the Drawings

The structure of the present invention will be described in detail below in combination with the accompanying drawings:

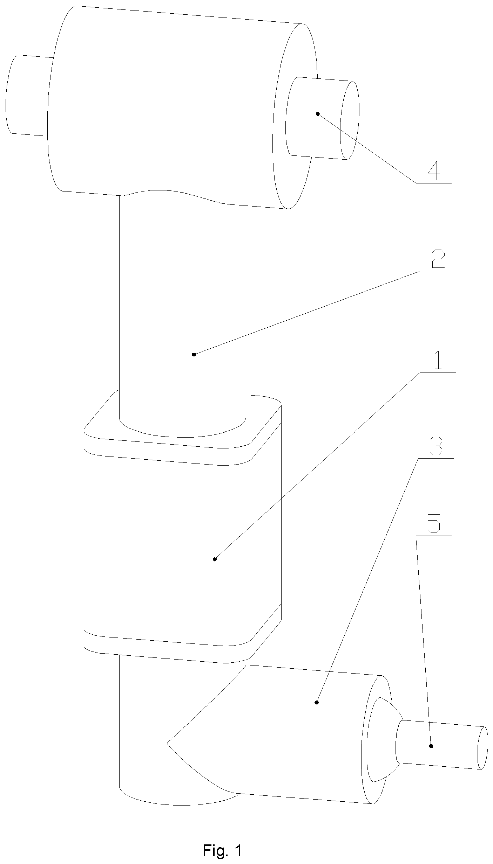

Fig. 1 illustrates a three-dimensional appearance diagram of an electric locking rotary joint.

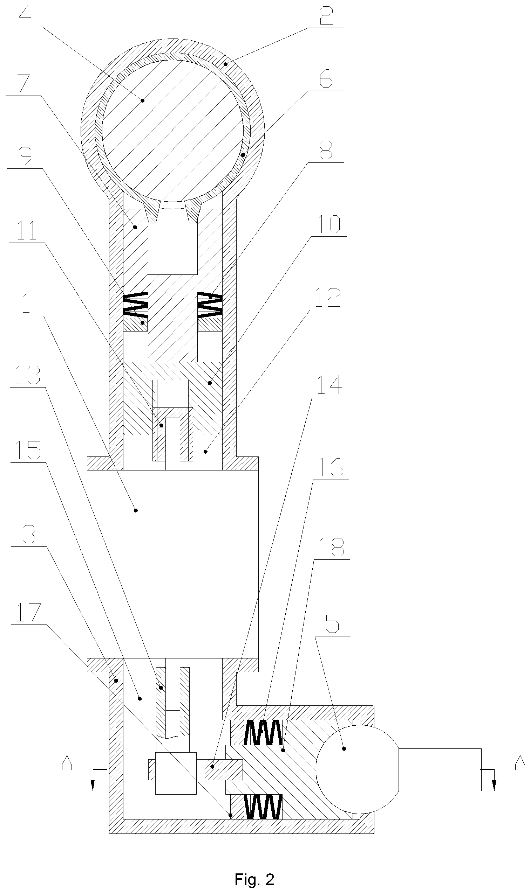

Fig. 2 illustrates a cross-sectional view of the electric locking rotary joint.

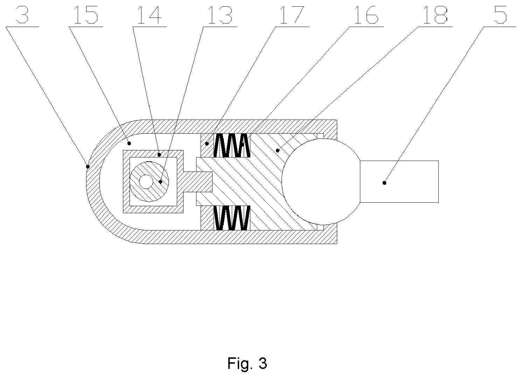

Fig. 3 illustrates an A-A cross-sectional view in Fig. 2.

1. motor; 2. shaft joint shell; 3. ball joint shell; 4. rotating shaft; 5. ball head; 6. brake ring; 7. locking fork; 8. disc spring set P; 9. baffle P; 10. nut; 11. screw; 12. space T; 13. plate cam; 14. flat-faced follower; 15. space L; 16. disc spring set Q; 17. baffle Q; 18. ball head pressing block.

### Detailed Description of the Embodiments

As shown in the drawings, the electric locking rotary joint is mainly includes a motor, a shaft joint and a ball joint, the motor 1 is a double-output-shaft motor or a single-output-shaft motor, corresponding to each motor shaft, a shaft joint shell 2 or a ball joint shell 3 is fixedly connected with the machine body of the motor 1, and meanwhile, a locking and loosening mechanism of the shaft joint or the ball joint is fixedly connected with the motor shaft.

The specific structure of the shaft joint is: the shaft joint shell 2 is rotatably sleeved on a rotating shaft 4, a axis of the rotating shaft 4 is perpendicular to a axis of the motor 1, a brake ring 6 with an opening is sleeved on the rotating shaft 4, the two ends of the rotating shaft 4 extend out of the shaft joint shell 2, the rotating shaft 4 and the shaft joint shell 2 form the shaft joint, and the shaft joint shell 2 is hollow and is referred to as a space T 12, the shaft of the motor 1 is disposed in the space T 12, a screw 11 is fixed on the shaft of the motor 1, a nut 10 is disposed on the screw 11 to form a screw pair, a rear end of a locking fork 7 is fixedly connected with the nut 10, a front end of the locking fork abuts against the outer side of the opening of the brake ring 6, a baffle P 9 is fixed on the shaft joint shell 2 in the space T 12, one end face of a disc spring set P 8 is pressed on the baffle P 9, and the other end face of the disc spring set P 8 is pressed on the locking fork 7, so as to press the locking fork 7 towards the brake ring 6.

The specific structure of the ball joint is: the ball joint shell 3 is hollow and is referred to as a space L 15, the space L 15 includes a pore channel, and a segment of the pore channel includes an opening, a axis of the pore channel is perpendicular to a axis of the motor 1, a segment of spherical surface is disposed on the opening of the pore channel, a spherical pressing block 18 is disposed in the pore channel, the spherical surface of the spherical pressing block 18 and the spherical surface at the outlet of the pore channel form a spherical space, a ball head 5 is rotatably disposed in the space, the ball head 5 and the two spherical surfaces form the ball joint, a plate cam 13 fixed on the shaft of the motor 1 is disposed in the space L 15, a axis of the plate cam 13 is parallel to the axis of the motor 1, a flat-faced follower 14 is in contact coupling with the plate cam 13, the flat-faced follower 14 and the plate cam 13 form a cam pair, the flat-faced follower 14 is fixedly connected with the spherical pressing block 18, a baffle Q 17 fixed with the ball joint shell 3 is disposed in the space L 15, one end face of a disc spring set Q 16 is pressed on the baffle Q 17, and the other end face of the disc spring set Q 16 is pressed on the spherical pressing block 18, so as to press the spherical pressing block 18 towards the ball head 5.

In some embodiments, the brake ring 6 is an annular member with an opening, which is movably sleeved on the rotating shaft; two bosses which are outward are disposed on the opening, outer sides of the two bosses are inclined surfaces, the two inclined surfaces are symmetrical, the distance between the outer ends of the two inclined surfaces is small, and the distance between the inner ends is large to form an outer inclined surface group. The front end of the locking fork 9 is a group of inner inclined surfaces, and the geometric dimension of the group of inner inclined surfaces enables the same to be attached to the outer inclined surface group on the bosses of the brake ring 6. the spherical diameter at the opening of the pore channel in the space L 15, the spherical diameter of the spherical pressing block 18 and the diameter of the ball head 5 are the same.

The foregoing is merely an embodiment of a combination of the present invention, and is not intended to limit the present invention, and any modifications, equivalent replacements, improvements and the like, made within the ideas and principles of the present invention, are all included within the protection scope of the present invention.

## Claims

1. An electric locking rotary joint, mainly comprises a motor, a shaft joint and a ball joint, wherein the motor is a double-output-shaft motor or a single-output-shaft motor, corresponding to each motor shaft, the shaft joint shell or a ball joint shell is fixedly connected with the machine body of the motor, and meanwhile, a locking and loosening mechanism of the shaft joint or the ball joint is fixedly connected with the motor shaft.

2. The electric locking rotary joint as claimed in claim 1, wherein the shaft joint shell is rotatably sleeved on a rotating shaft, a axis of the rotating shaft is perpendicular to a axis of the motor shaft, a brake ring which comprises an opening is sleeved on the rotating shaft, the two ends of the rotating shaft extend out of the shaft joint shell, the rotating shaft and the shaft joint shell form the shaft joint, the shaft joint shell is hollow and is referred to as a space T, the motor shaft is disposed in the space T, a screw is fixed on the motor shaft, a nut is disposed on the screw to form a screw pair, a rear end of a locking fork is fixedly connected with the nut, a front end of the locking fork abuts against the outer side of the opening of the brake ring, a baffle P is fixed on the shaft joint shell in the space T, one end face of a disc spring set P is pressed on the baffle P, and the other end face of the disc spring set P is pressed on the locking fork, so as to press the locking fork towards the brake ring.

3. The electric locking rotary joint as claimed in claim 1, wherein the ball joint shell is hollow and is referred to as a space L, the motor shaft is disposed in the space L, the space L comprises a pore channel and a segment of the pore channel comprises an opening, a axis of the pore channel is perpendicular to a axis of the motor shaft, a segment of spherical surface is disposed on the opening of the pore channel, a spherical pressing block is disposed in the pore channel, the spherical surface of the spherical pressing block and the spherical surface at the outlet of the pore channel form a spherical space, a ball head is rotatably disposed in the space, the ball head and the two spherical surfaces form the ball joint, a plate cam fixed on the motor shaft is disposed in the space L, a axis of the plate cam is parallel to the axis of the motor shaft, a flat-faced follower is in contact coupling with the plate cam, the flat-faced follower and the plate cam form a cam pair, the flat-faced follower is fixedly connected with the spherical pressing block, a baffle Q fixed with the ball joint shell is disposed in the space L, one end face of a disc spring set Q is pressed on the baffle Q, and the other end face of the disc spring set Q is pressed on the spherical pressing block, so as to press the spherical pressing block towards the ball head.

4. The electric locking rotary joint as claimed in claim 2, wherein the brake ring is an annular member with an opening, which is movably sleeved on the rotating shaft; two bosses which are outward are disposed on the opening, outer sides of the two bosses are inclined surfaces, the two inclined surfaces are symmetrical, the distance between the outer ends of the two inclined surfaces is small, and the distance between the inner ends is large to form an outer inclined surface group.

5. The electric locking rotary joint as claimed in claim 2, wherein the front end of the locking fork is a group of inner inclined surfaces, and the geometric dimension of the group of inner inclined surfaces enables the same to be attached to the outer inclined surface group on the bosses of the brake ring.

6. The electric locking rotary joint as claimed in claim 3, wherein the spherical diameter at the opening of the open pore channel in the space L, the spherical diameter of the spherical pressing block and the diameter of the ball head are the same.

7. The electric locking rotary joint as claimed in claim 1, wherein the motor is a servo motor or a stepper motor.
